# FASCICULE DE BREVET EUROPEEN

(11) **EP 1 371 659 B1**
(45) Date de publication et mention de la délivrance du brevet: **12.10.2005**
(21) Numéro de dépôt: 03292133.0
(22) Date de dépôt: 29.08.2003
(51) Int. Cl.: C07K 5/06, C07K 5/02, C07D 209/42

(54) **Nouveau procédé de synthèse du perindopril et de ses sels pharmaceutiquement acceptables**
Verfahren für die Synthese von Perindopril und seiner pharmazeutischen annehmbaren Salzen
Method for synthesis of perindopril and its pharmaceutically acceptable salts [2003/26]

(43) Date de publication de la demande: 17.12.2003
(73) Titulaire: Les Laboratoires Servier, 92415 Courbevoie Cedex (FR)
(72) Inventeur: Dubuffet, Thierry, 76190 Autretot (FR); Lecouve, Jean-Pierre, 76600 Le Havre (FR)

(56) Documents cités:
- EP-A- 1 319 668
- HUFFMAN M A ET AL: "Improved Stereoselectivity in the Heterogeneous Catalytic Synthesis of Enalapril Obtained Through Multidimensional Screening" TETRAHEDRON LETTERS, ELSEVIER SCIENCE PUBLISHERS, AMSTERDAM, NL, vol. 40, no. 5, 29 janvier 1999 (1999-01-29), pages 831-834, XP004151458 ISSN: 0040-4039
- BLACKLOCK T J ET AL: "SYNTHESIS OF SEMISYNTHETIC DIPEPTIDES USING N-CARBOXYANHYDRIDES ANDCHIRAL INDUCTION ON RANEY NICKEL. A METHOD PRACTICAL FOR LARGE SCALE" JOURNAL OF ORGANIC CHEMISTRY, AMERICAN CHEMICAL SOCIETY. EASTON, US, vol. 53, no. 4, 1988, pages 836-844, XP000914389 ISSN: 0022-3263

## Description

La présente invention concerne un procédé de synthèse industrielle du perindopril de formule (I): 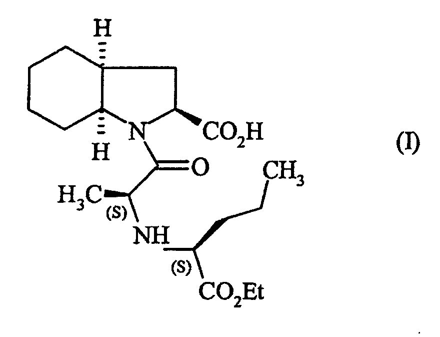 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse industrielle performant, facilement transposable à l'échelle industrielle, conduisant au perindopril avec un bon rendement et une excellente pureté, à partir de matières premières bon marché.
Le brevet EP 0 308 341 décrit la synthèse industrielle du perindopril par couplage de l'ester benzylique de l'acide (2*S*, 3a*S*, 7a*S*)-octahydroindole 2-carboxylique avec l'ester éthylique de la N-[(S)-1-carboxybutyl]-(S)-alanine, suivie de la déprotection du groupement carboxylique de l'hétérocycle par hydrogénation catalytique.

La demanderesse a présentement mis au point un nouveau procédé de synthèse du perindopril.

Plus spécifiquement, la présente invention concerne un procédé de synthèse industrielle du perindopril, et de ses sels pharmaceutiquement acceptables, caractérisé en ce que l'on fait réagir le composé de formule (II) : 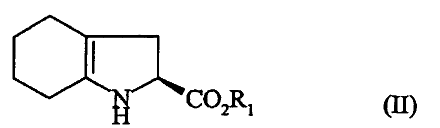 dans laquelle R₁ représente un groupement benzyle ou alkyle (C₁-C₆) linéaire ou ramifié,
avec un composé de formule (III), de configuration S : 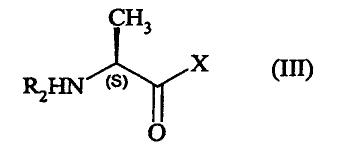 dans laquelle X représente un atome d'halogène, et R₂ représente un groupement protecteur de la fonction amino,
en présence d'une base,
pour conduire, après déprotection de la fonction amino, au composé de formule (IV) : 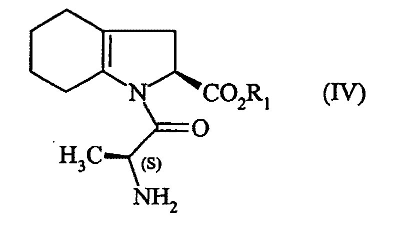 dans laquelle R₁ est tel que défini précédemment,
que l'on met en réaction avec le 2-oxo-pentanoate d'éthyle, sous pression d'hydrogène, en présence de platine sur charbon,
pour conduire au composé de formule (V) : 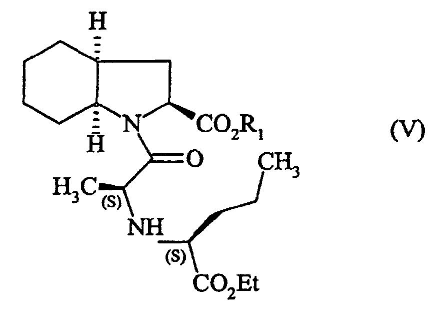 dans laquelle R₁ est tel que défini précédemment,
que l'on déprotège, pour conduire au composé de formule (I).

Parmi les groupements protecteurs de la fonction amino utilisables dans le procédé de la présente invention, on peut citer à titre non limitatif les groupements tert-butyloxycarbonyle, benzyloxycarbonyle et benzyle.

R₁ représente préférentiellement le groupement benzyle. Dans ce cas, le groupement protecteur de la fonction amino est préférentiellement le groupement tert-butyloxycarbonyle.

Parmi les bases utilisables dans la réaction entre les composés de formules (II) et (III), on peut citer à titre non limitatif les amines organiques telles que la triéthylamine, la pyridine, la N-méthylmorpholine ou la diisopropyléthylamine, et les bases minérales telles que NaOH, KOH, Na₂CO₃, K₂CO₃, NaHCO₃ ou KHCO₃.

La réaction entre le composé de formule (IV) et le 2-oxopentanoate d'éthyle est préférentiellement effectuée dans l'acétate d'éthyle, l'acide acétique ou dans un solvant alcoolique, sous pression atmosphérique, à une température comprise entre 20 et 60°C.

### EXEMPLE : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : (2S)-1-{(2S)-2-[(Tert-butyloxycarbonyl)-amino]-propionyl}-2,3,4,5,6,7-hexahydro-1H-indole-2-carboxylate de benzyle

Dans un réacteur, charger 200 g de (2S)-2,3,4,5,6,7-hexahydro-1H-indole-2-carboxylate de benzyle, 1,5 1 de dichlorométhane, puis amener la température du mélange réactionnel à 0°C et ajouter 107 ml de triéthylamine, puis 162 g de chlorure de (2S)-2-[(tert-butyloxycarbonyl)-amino]-propionyle. Amener ensuite le mélange à température ambiante. Après 1h d'agitation à cette température, laver le mélange à l'eau puis avec une solution diluée d'acide acétique. La solution de (2S)-1-{(2S)-2-[(tert-butyloxycarbonyl)-amino]-propionyl}-2,3,4,5,6,7-hexahydro-1*H*-indole-2-carboxylate de benzyle ainsi obtenue est engagée telle quelle dans l'étape suivante.

### Stade B : (2S)-1-{(2S)-2-Aminopropionyl}-2,3,4,5,6,7-hexahydro-1H-indole-2-carboxylate de benzyle

Dans un réacteur, charger la solution obtenue au stade précédent, puis ajouter 133 g d'acide trifluoroacétique. Après 1h30 d'agitation à température ambiante, laver le mélange à l'eau, puis avec une solution saturée de bicarbonate de soude, et évaporer les solvants, pour conduire au (2S)-1-{(2S)-2-aminopropionyl}-2,3,4,5,6,7-hexahydro-1*H*-indole-2-carboxylate de benzyle.

### Stade C : (2S,3aS,7aS)-1-{(2S)-2-[(1S)-1-(Ethoxycarbonyl)-butylamino]-propionyl}-octahydro-1H-indole-2-carboxylate de benzyle

Dans un hydrogénateur, placer 200 g du composé obtenu dans le stade précédent et 88 g de 2-oxopentanoate d'éthyle en solution dans l'acétate d'éthyle, puis 5 g de Pt/C à 10 %. Hydrogéner sous pression atmosphérique à 40°C, jusqu'à absorption de la quantité théorique d'hydrogène.
Eliminer le catalyseur par filtration, puis refroidir entre 0 et 5°C et récolter le solide obtenu par filtration, laver le gâteau et le sécher jusqu'à poids constant.
Le (2S, 3aS, 7aS)-1-{(2S)-2-[(1S)-1-(éthoxycarbonyl)-butylamino]-propionyl}-octahydro-1*H*-indole-2-carboxylate de benzyle est ainsi obtenu avec un rendement de 85 %.

### Stade D : Acide (2S, 3aS, 7aS)-1-{(2S)-2-[(1S)-1-(éthoxycarbonyl)-butylamino]-propionyl}-octahydro-1H-indole-2-carboxylique

Dans un hydrogénateur, placer 200 g du composé obtenu dans le stade précédent en solution dans l'éthanol, puis 5 g de Pd/C à 10 %. Hydrogéner sous pression atmosphérique à 30°C, jusqu'à absorption de la quantité théorique d'hydrogène.
Eliminer le catalyseur par filtration, puis évaporer le solvant.
L'acide (2S, 3aS, 7aS)-1-{(2S)-2-[(1S)-1-(éthoxycarbonyl)-butylamino]-propionyl}-octahydro-1*H*-indole-2-carboxylique est ainsi obtenu avec un rendement de 85 %.

### Stade E : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

Le composé obtenu dans le stade précédent (200 g) est mis en solution dans 2,8 1 d'acétonitrile, puis 40 g de tert-butylamine et 0,4 1 d'acétate d'éthyle sont ajoutés.
La suspension obtenue est ensuite portée au reflux jusqu'à dissolution totale, puis la solution obtenue est filtrée à chaud et refroidie sous agitation jusqu'à une température de 15-20°C. Le précipité obtenu est alors filtré, réempâté à l'acétonitrile, séché puis recristallisé dans l'acétate d'éthyle pour conduire au produit attendu avec un rendement de 95% et une pureté énantiomérique de 99%.

## Revendications

1. Procédé de synthèse industrielle des composés de formule (I): 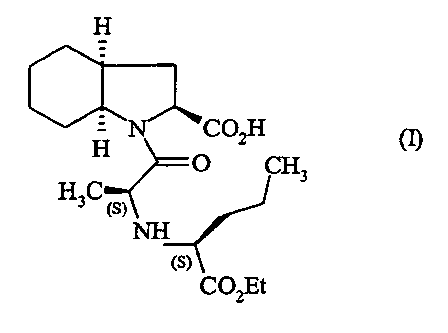 et de ses sels pharmaceutiquement acceptables,
**caractérisé en ce que** l'on fait réagir le composé de formule (II) : 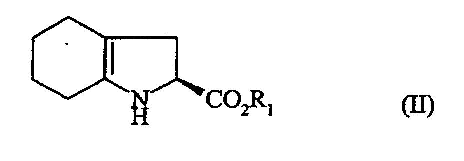 dans laquelle R₁ représente un groupement benzyle ou alkyle (C₁-C₆) linéaire ou ramifié,
avec un composé de formule (III), de configuration S : 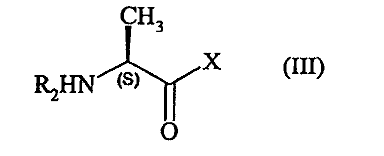 dans laquelle X représente un atome d'halogène, et R₂ représente un groupement protecteur de la fonction amino,
en présence d'une base,
pour conduire, après déprotection de la fonction amino, au composé de formule (IV) : 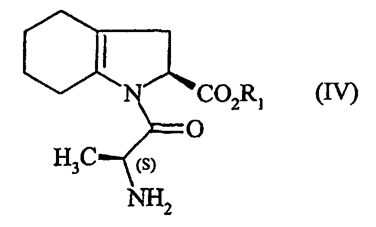 dans laquelle R₁ est tel que défini précédemment,
que l'on met en réaction avec le 2-oxo-pentanoate d'éthyle, sous pression d'hydrogène, en présence de platine sur charbon,
pour conduire au composé de formule (V) : 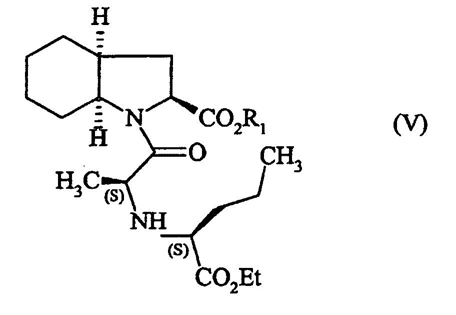 dans laquelle R₁ est tel que défini précédemment,
que l'on déprotège, pour conduire au composé de formule (I).

2. Procédé de synthèse selon la revendication 1, **caractérisé en ce que** le groupement protecteur de la fonction amino est un groupement tert-butyloxycarbonyle, benzyloxycarbonyle ou benzyle.

3. Procédé de synthèse selon la revendication 2, **caractérisé en ce que** R₁ représente un groupement benzyle, et le groupement protecteur de la fonction amino est un groupement tert-butyloxycarbonyle.

4. Procédé de synthèse selon l'une quelconque des revendications 1 à 3, **caractérisé en ce que** la base utilisée pour la réaction entre les composés de formules (II) et (III) est une amine organique choisie parmi la triéthylamine, la pyridine, la N-méthylmorpholine et la diisopropyléthylamine, ou une base minérale telle que NaOH, KOH, Na₂CO₃, K₂CO₃, NaHCO₃ ou KHCO₃.

5. Procédé de synthèse selon l'une quelconque des revendications 1 à 4, **caractérisé en ce que** la réaction entre le composé de formule (IV) et le 2-oxopentanoate d'éthyle est effectuée dans l'acétate d'éthyle, l'acide acétique ou dans un solvant alcoolique.

6. Procédé de synthèse selon l'une quelconque des revendications 1 à 5, **caractérisé en ce que** la réaction entre le composé de formule (IV) et le 2-oxopentanoate d'éthyle est effectuée à pression atmosphérique.

7. Procédé de synthèse selon l'une quelconque des revendications 1 à 6, **caractérisé en ce que** la réaction entre le composé de formule (IV) et le 2-oxopentanoate d'éthyle est effectuée à une température comprise entre 20 et 60°C.

8. Procédé de synthèse selon l'une quelconque des revendications 1 à 7 du perindopril sous sa forme de sel de tert-butylamine.

## Patentansprüche

1. Verfahren zur technischen Synthese der Verbindung der Formel (I): 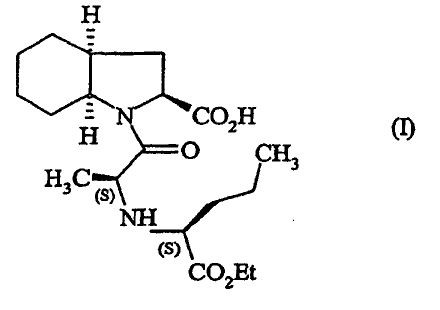 und von ihren pharmazeutisch annehmbaren Salzen,
**dadurch gekennzeichnet, daß** man die Verbindung der Formel (II): 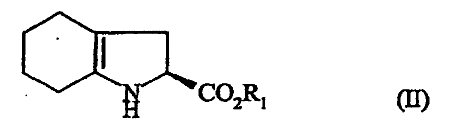 in der R₁ eine Benzylgruppe oder eine geradkettige oder verzweigte (C₁-C₆)-Alkylgruppe darstellt,
mit einer Verbindung der Formel (III) in der Konfiguration S: 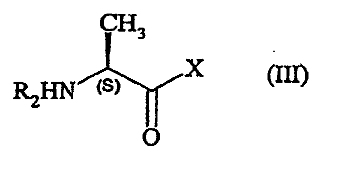 in der X ein Halogenatom und R₂ eine Schutzgruppe für die Aminofunktion bedeuten,
in Gegenwart einer Base umsetzt,
so daß man nach der Abspaltung der Schutzgruppe der Aminofunktion die Verbindung der Formel (IV) erhält: 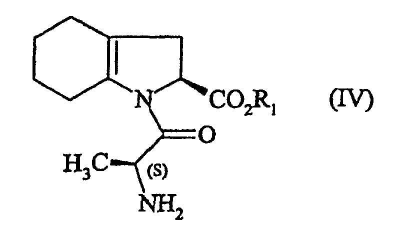 worin R₁ die oben angegebenen Bedeutungen besitzt,
welche man in Gegenwart von Platin-auf-Kohlenstoff unter Wasserstoffdruck mit 2-Oxo-pentansäureethylester umsetzt,
zur Bildung der Verbindung der Formel (V): 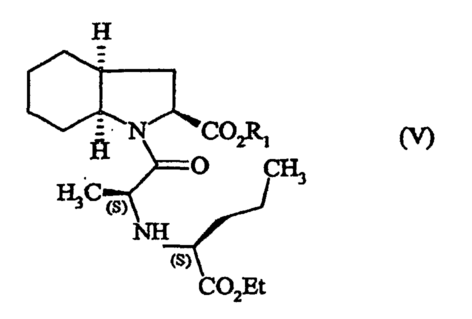 in der R₁ die oben angegebenen Bedeutungen besitzt,
von welcher man die Schutzgruppe abspaltet zur Bildung der Verbindung der Formel (I).

2. Syntheseverfahren nach Anspruch 1, **dadurch gekennzeichnet, daß** die Schutzgruppe der Aminofunktion eine tert.-Butyloxycarbonylgruppe, Benzyloxycarbonylgruppe oder Benzylgruppe ist.

3. Syntheseverfahren nach Anspruch 2, **dadurch gekennzeichnet, daß** R₁ eine Benzylgruppe bedeutet und die Schutzgruppe für die Aminofunktion eine tert.-Butyloxycarbonylgruppe bedeutet.

4. Syntheseverfahren nach einem der Ansprüche 1 bis 3, **dadurch gekennzeichnet, daß** die für die Reaktion der Verbindungen der Formeln (II) und (III) verwendete Base ein organisches Amin ausgewählt aus Triethylamin, Pyridin, N-Methylmorpholin und Diisopropylethylamin oder eine anorganische Base, wie NaOH, KOH, Na₂CO₃, K₂CO₃, NaHCO₃ oder KHCO₃ ist.

5. Syntheseverfahren nach einem der Ansprüche 1 bis 4, **dadurch gekennzeichnet, daß** die Umsetzung der Verbindung der Formel (IV) mit 2-Oxopentansäureethylester in Ethylacetat, Essigsäure oder einem alkoholischen Lösungsmittel durchgeführt wird.

6. Syntheseverfahren nach einem der Ansprüche 1 bis 5, **dadurch gekennzeichnet, daß** die Umsetzung der Verbindung der Formel (IV) mit dem 2-Oxo-pentansäureethylester bei Atmosphärendruck durchgeführt wird.

7. Syntheseverfahren nach einem der Ansprüche 1 bis 6, **dadurch gekennzeichnet, daß** die Umsetzung der Verbindung der Formel (IV) mit dem 2-Oxo-pentansäureethylester bei einer Temperatur zwischen 20 und 60°C durchgeführt wird.

8. Verfahren nach einem der Ansprüche 1 bis 7 zur Synthese von Perindopril in Form seines tert.-Butylaminsalzes.

## Claims

1. Process for the industrial synthesis of the compounds of formula (I) : 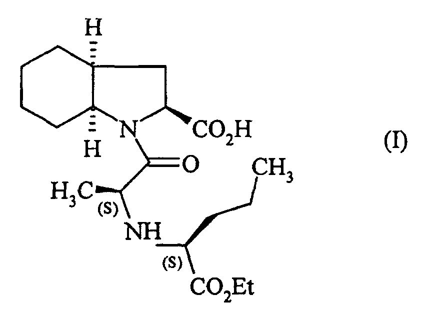 and its pharmaceutically acceptable salts,
**characterised in that** a compound of formula (II) : 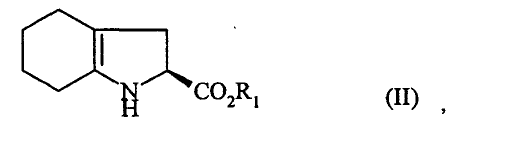 wherein R₁ represents a benzyl or linear or branched (C₁-C₆)alkyl group,
is reacted with a compound of formula (III) having the S configuration : 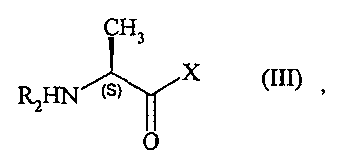 wherein X represents a halogen atom and R₂ represents a protecting group for the amino function,
in the presence of a base,
to yield, after deprotection of the amino function, a compound of formula (IV) : 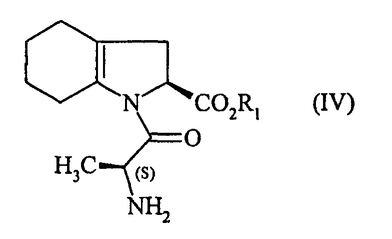 wherein R₁ is as defined hereinbefore,
which is reacted with ethyl 2-oxopentanoate under hydrogen pressure, in the presence of platinum-on-carbon,
to yield a compound of formula (V) : 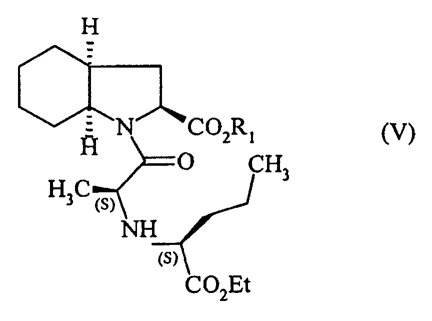 wherein R₁ is as defined hereinbefore,
which is deprotected to yield the compound of formula (I).

2. Synthesis process according to claim 1, **characterised in that** the protecting group for the amino function is a tert-butoxycarbonyl, benzyloxycarbonyl or benzyl group.

3. Synthesis process according to claim 2, **characterised in that** R₁ represents a benzyl group and the protecting group for the amino function is a tert-butoxycarbonyl group.

4. Synthesis process according to any one of claims 1 to 3, **characterised in that** the base used for the reaction between the compounds of formulae (II) and (III) is an organic amine selected from triethylamine, pyridine, N-methylmorpholine and diisopropylethylamine, or a mineral base such as NaOH, KOH, Na₂CO₃, K₂CO₃, NaHCO₃ or KHCO₃.

5. Synthesis process according to any one of claims 1 to 4, **characterised in that** the reaction between the compound of formula (IV) and ethyl 2-oxopentanoate is carried out in ethyl acetate, acetic acid or in an alcoholic solvent.

6. Synthesis process according to any one of claims 1 to 5, **characterised in that** the reaction between the compound of formula (IV) and ethyl 2-oxopentanoate is carried out at atmospheric pressure.

7. Synthesis process according to any one of claims 1 to 6, **characterised in that** the reaction between the compound of formula (IV) and ethyl 2-oxopentanoate is carried out at a temperature of from 20 to 60°C.

8. Process according to any one of claims 1 to 7 for the synthesis of perindopril in the form of its tert-butylamine salt.
